# FASCICULE DE BREVET EUROPEEN

(11) **EP 1 101 116 B1**
(45) Date de publication et mention de la délivrance du brevet: **02.10.2002**
(21) Numéro de dépôt: 99934803.0
(22) Date de dépôt: 28.07.1999
(51) Int. Cl.: G01N 33/543, G01N 33/52

(54) **DISPOSITIF ET PROCEDE ELECTROSTATIQUES DE DETECTION IMMUNOLOGIQUE**
VORRICHTUNG UND VERFAHREN ZUR ELEKTROSTASTISCHEN IMMUNOLOGISCHEN BESTIMMUNG
ELECTROSTATIC DEVICE AND METHOD FOR IMMUNOLOGICAL DETECTION

(30) Priorité: 28.07.1998 FR 9809599
(43) Date de publication de la demande: 23.05.2001
(73) Titulaire: Infrabio, 78160 Marly le Roi (FR)
(72) Inventeur: TOLEDANO, Jacques, F-75020 Paris (FR)
(74) Mandataire: Dawidowicz, Armand
(86) Numéro de dépôt international: FR9901868
(87) Numéro de publication internationale: WO00007020

(56) Documents cités:
- WO-A-92/06380
- US-A- 4 948 726
- CHEMICAL ABSTRACTS, vol. 116, no. 17, 27 avril 1992 (1992-04-27) Columbus, Ohio, US; abstract no. 171745, HUANG, PING YU ET AL: "Mechanistic studies of electrostatic potentials on antigen- antibody complexes for bioanalyses" XP002101860 & ANAL. CHEM. (1992), 64(9), 977-80 CODEN: ANCHAM;ISSN: 0003-2700,

## Description

Les dispositifs et procédés de l'art antérieur pour la réalisation de diagnostics immunologiques précis mettent généralement en oeuvre des dispositifs ayant le plus souvent déjà fait l'objet d'une préparation immunologique (par immobilisation d'un premier anticorps). Ainsi les puits des plaques mises en oeuvre dans les procédés du type ELISA comportent en général un revêtement comprenant des premiers anticorps pour capter un antigène, lequel sera reconnu ensuite par un second anticorps, le tout étant révélé ensuite par un troisième anticorps anti-immunoglobulines. Une bonne exécution de ce procédé requiert au moins six lavages et trois heures d'incubation.

Les tests rapides apparus dans les années 80 utilisent des microbilles préalablement revêtues par un premier anticorps et qui migrent sur une surface de nylon pour s'arrêter éventuellement sur une première ligne (présence d'antigènes) de seconds anticorps, avant de s'arrêter en tous cas sur une deuxième ligne de troisièmes anticorps. Le tout se fait en plusieurs minutes, ne dose qu'un minimum de 7 nanogrammes d'antigène par ml d'échantillon. Ce procédé ne permet pas de détecter les anticorps sériques.

L'objet de la présente invention n'est pas non plus une électrophorèse qui se caractérise par une migration plus ou moins longue entre deux pôles électriques (l'un au départ et l'autre à l'arrivée), en fonction du poids moléculaire, de protéines différentes. Il existe des techniques plus ou moins sophistiquées de gel pris entre deux électrodes et qui permettent de séparer un groupe de protéines en bandes différentes selon leur poids moléculaire.

La présente invention a pour but de remédier aux insuffisances de sensibilité des procédés antérieurs, en particulier de fournir un procédé permettant d'obtenir un signal qualitatif ou quantitatif de la présence, à des doses infinitésimales, d'une substance dans les fluides d'un échantillon, même si elle n'est pas constituée par un acide nucléique. La technique dénommée PCR (Polymerase Chain Reaction) parvient à détecter des quantités infimes de fragments de matériel chromosomique (ARN ou ADN). Elle n'est pas capable de doser les protéines comme les nouvelles générations de marqueurs tumoraux ou de cytokines circulant à des taux qui peuvent être inférieurs au picogramme par millilitre (un milliardième de gramme). Les techniques immunologiques, même les plus sensibles comme la radio-immuno-analyse ne le permettent pas.

Le procédé selon l'invention est applicable à la détection de tout analyte éventuellement contenu ou susceptible d'être entraîné dans un fluide ou liquide qui peut réagir avec un ligand spécifique pour former un complexe, l'analyte ou le ligand ou les deux à la fois étant porteurs de charges électriques qui sont neutralisées, voire même inversées dans le complexe lorsque celui-ci est formé. Il est caractérisé en ce que :
- l'on fait migrer ce fluide ou liquide, après sa mise en contact avec le ligand spécifique, dans une matière poreuse électriquement inerte intercalée entre les faces opposées de deux électrodes électriquement chargées, de même polarité, et
- l'on détecte dans le liquide ayant migré dans la matière poreuse entre les deux électrodes, ceux des composants, analyte, ligand ou complexe qui n'ont pas été captés par les électrodes.

Il va sans dire que le volume total de fluide ou liquide mis en oeuvre, qu'il s'agisse de celui qui contient à l'état dissous l'un des susdits constituants ou les deux à la fois, notamment à l'issue de leur mélange éventuel, doit être suffisant pour autoriser sa diffusion dans la matière poreuse jusqu'à l'endroit où est réalisée la détection.

De la nature du constituant détecté -ou non détecté- dépend alors la conclusion que l'on peut tirer du dosage. Dans une forme préférée du procédé selon l'invention, l'un des constituants, par exemple le ligand, est marqué. S'il s'agit par exemple d'un anticorps qui porte des groupes libres NH₂, donc des charges positives (l'analyte étant alors un antigène), et si les électrodes sont chargées négativement, la détection du marqueur dans le fluide ou liquide ayant migré dans la matière poreuse entre les électrodes attestera de ce que l'anticorps est engagé dans un complexe, donc que l'antigène était présent dans le liquide ou fluide d'origine. En effet à la suite de la réaction antigène-anticorps, les groupes NH₂ de l'anticorps sont masqués dans le complexe formé, la charge positive de l'anticorps étant alors neutralisée, le complexe antigène-anticorps pouvant même encore, le cas échéant, parfois présenter une polarité négative. Au contraire, l'absence de détection traduira l'absence de l'antigène dans le fluide ou le liquide étudié. En effet les anticorps marqués qui n'ont alors pas rencontré de « partenaires » sont alors captés et bloqués par les électrodes chargées négativement.

D'autres exemples témoignent dans la suite de cette description des importantes possibilités qu'offre l'invention. Mais pour la bonne compréhension de l'invention, il peut être utile de définir les expressions « analyte » et « ligand », telles qu'elles sont utilisées dans la présente description. En effet, l'application de l'invention n'est limitée à la détection d'analytes seulement constitués par des antigènes ou des anticorps, même si c'est à leur propos que l'invention sera le plus souvent mise en oeuvre.

Par analyte, il faut entendre toute molécule ou entité que l'on souhaite détecter, quelle que soit sa nature: haptène, protéine, antigène, anticorps, fragments d'acide nucléique, substance, voire d'un groupe de molécules répondant à des caractéristiques communes, cet analyte étant susceptible de donner lieu à une réaction spécifique avec un ligand pour former un complexe susceptible d'être mis en jeu dans une réaction visant à détecter de façon sélective la présence ou non de l'analyte dans un fluide ou liquide, par exemple un fluide d'origine biologique.

Dans le cas d'un analyte constitué par un antigène, on appréciera que l'anticorps, qui joue alors le rôle du ligand, est porteur de sites NH₂ qui lui confèrent une polarité positive, entraînant la possibilité pour cet anticorps à être capté par une électrode portant des charges négatives, que celles-ci soient de nature électrostatique ou le résultat de la mise sous tension de l'électrode dans le sens approprié. Naturellement l'inverse peut aussi se produire. Il peut se produire, par exemple, que dans d'autres types de couples analyte-ligand, le groupe polaire soit formé d'un groupe négativement chargé, par exemple un groupe COOH, qui serait neutralisé dans le complexe analyte-ligand formé.

Il va de soi, que le ligand peut au besoin être lui-même modifié par un groupe visant à lui conférer les propriétés, par exemple la polarité électrique requise pour sa mise en oeuvre dans le procédé de l'invention. Ce peut, à titre d'exemple, être le cas lorsque l'analyte est constitué par un fragment d'ADN, dont la présence peut être recherchée dans un liquide après qu'il ait lui-même été mis en oeuvre dans un essai de détection d'un ARN complémentaire. Dans un tel cas, le fragment d'ADN sera, par exemple, rendu porteur d'un groupe biotine, le ligand alors susceptible d'être utilisé pouvant être constitué par de l'avidine, portant elle-même, le cas échéant, un marqueur, par exemple colorimétrique, fluorescent chemiluminescent, radioactif ou autre et, s'il en était encore besoin, un groupe supplémentaire lui conférant une charge électrostatique, laquelle pourrait alors être neutralisée lors de la production du complexe analyte-ligand alors susceptible d'être formé, via la réaction biotine-avidine. Ou encore l'analyte peut être constitué par un fragment d'ADN et le ligand par une sonde d'ADN elle-même marquée par une enzyme, notamment de la peroxydase, par l'intermédiaire de bras espaceurs produits par l'intermédiaire de systèmes hétérobifonctionnels, par exemple du type (ADN (FMCC)-peroxydase (SPDP).

De préférence l'analyte est en solution dans un liquide ou un fluide. Mais, par exemple, lorsque l'analyte consiste en un antigène, il peut aussi être contenu, par exemple, dans un fragment de biopsie ou de muqueuse. Mais il peut alors être extrait par, ou entraîné dans, le liquide qui va ensuite migrer dans la matière poreuse entre les électrodes.

En ce qui concerne la matière « poreuse », il faut naturellement entendre sous cette expression toute matière permettant la propagation ou diffusion d'un liquide dans sa masse, lorsqu'elle vient à être mouillée par ce liquide. Elle peut être constituée de toute matière à porosité ouverte, matière fibreuse ou apte à se comporter comme un buvard, etc...

Pour ce qui est des électrodes, on peut avoir recours à tous matériaux aptes à être polarisés électriquement ou chargés électrostatiquement. De nombreuses matières sous forme de feuilles minces sont disponibles dans le commerce : voir par exemple le chapitre commençant à la page 53 du catalogue « DIMACEL composants et relatif aux emballages électrostatiques ».

L'homme du métier est évidemment à même d'imaginer nombre d'applications différentes de l'invention. Ce sera le cas pour toute détection d'une substance déterminée ou « analyte » mettant en oeuvre une réaction hautement spécifique avec un « ligand » correspondant. Il sera dans tous les cas capable, sans faire oeuvre inventive et si le besoin devait s'en faire sentir, de modifier l'un des constituants de la réaction pour lui conférer une polarité qui serait neutralisée, voire même inversée dans le produit de réaction final, pour qu'en définitive le procédé de l'invention puisse également lui être appliqué. De même l'homme du métier sera dans chaque cas capable d'adapter le procédé de l'invention, par exemple de choisir les polarités à conférer aux électrodes pour le rendre opérationnel pour la détection de tout analyte susceptible de former un complexe spécifique avec un ligand donné, lorsque cette formation s'accompagne aussi d'une neutralisation, voire inversion de la charge électrique, soit de l'analyte, soit du ligand.

Donc d'une façon plus générale, lorsque les polarités des charges portées par le ligand et celles des électrodes sont de signe contraire, l'on détecte le complexe dans le liquide ayant migré entre les deux électrodes lorsque le liquide initial contenait l'analyte.

En revanche, lorsque les polarités des charges portées par le ligand et celle des électrodes sont de même signe et les polarités des charges portées par l'analyte et le complexe lui-même sont de signe contraire, l'on ne détecte que l'excédent éventuel du ligand initialement mis en oeuvre lorsque l'analyte était initialement présent.

Comme cela a déjà été mentionné, il est avantageux que le ligand porte un marqueur, en particulier un marqueur transformable en un signal colorimétrique, fluorescent ou chemiluminescent et que la zone-signal comprenne un révélateur du marqueur, dans la zone-signal à laquelle accède le liquide ayant migré dans la matière poreuse, entre les électrodes.

La zone-signal est elle-même avantageusement formée dans une matière poreuse en contact avec celle qui est intercalée entre les électrodes, de façon à autoriser l'accès dans la zone-signal du liquide ayant migré d'abord dans la matière poreuse intercalée entre les électrodes, puis dans la matière poreuse comportant la zone-signal.

Dans l'un de ses modes de mise en oeuvre préférés, le procédé objet de la présente invention met donc en oeuvre l'attraction ou la répulsion électrique exercée par deux ou plusieurs électrodes (de préférence minces et planes), sur l'un des constituants du couple analyte-ligand, par exemple sur des immunoglobulines (lg) selon que ces immunoglobulines sont liées ou non à leur antigène spécifique. Une micro-goutte d'échantillon à tester est déposée sur la matière poreuse, elle-même généralement formée d'une feuille mince, à l'amont de celle-ci ou dans la région amont de la paire d'électrodes, la goutte d'échantillon étant alors absorbée immédiatement par la feuille poreuse, souvent un papier isolant. Puis on dépose un volume de solution d'anticorps marqués qui vont se mélanger à la goutte d'échantillon préalablement déposée. Si l'antigène recherché est présent dans la goutte d'échantillon, il va être rapidement capté par les anticorps marqués. Les électrodes vont alors faire la différence entre un anticorps complexé à l'antigène et un anticorps qui n'a pas rencontré d'antigène dans la gouttelette, en particulier lorsqu'elles sont parcourues par un courant électrique négatif. Dans ce cas, les électrodes captent les sites NH₂ (chargé positivement) des immunoglobulines qui ne se sont pas liées à un antigène, et laissent migrer les immunoglobulines (Ig) dont le site NH₂ est cette fois saturé et masqué par un antigène dans la feuille poreuse, entre les deux électrodes, jusque dans une zone-signal dans laquelle elles peuvent être détectées et mesurées, lorsqu'elles ont été préalablement préparées avant le dosage (conjuguées à une enzyme ou un marqueur radioactif ou tout autre moyen).

Mais ces électrodes peuvent également être chargées positivement ou être parcourues par un courant positif, repoussant cette fois-ci les sites NH₂ des immunoglobulines (Ig) restés libres de toute liaison à un antigène. Elles vont par contre capter les complexes lg + antigènes qui présentent tous un nombre suffisant de charges négatives, alors captées par cette électrode positive. Il se produit donc une inversion du signal à la fin du dosage : le signal n'est fourni que par les lg restées libres de tout antigène.

On peut avoir recours à des combinaisons de paires d'électrodes associées avec la matière poreuse, en particulier lorsqu'elle est prise en sandwich successivement entre une première paire puis, en aval de celle-ci, avec une seconde paire d'électrodes, les électrodes de cette deuxième paire étant également chargées et de polarités identiques entre elles, mais de signe contraire à celui des polarités des électrodes de la précédente paire, la détection étant alors effectuée en amont de la deuxième paire d'électrodes, après que le liquide ait migré dans la matière poreuse d'abord à travers la première puis à travers la seconde paire d'électrodes. Cette procédure permet alors aussi des dosages semi-quantitatifs, dès lors que la première paire d'électrodes ne peut retenir qu'une quantité déterminée selon le cas, soit du ligand marqué, soit du complexe formé, la détection finale portant alors seulement sur l'excédent selon le cas, soit du complexe formé, soit de l'analyte initialement présent dans le fluide ou liquide étudié.

Avantageusement la première paire d'électrodes est d'un signe tel et est calibrée de façon à piéger une quantité maximum prédéterminée de l'analyte sous forme du complexe analyte-ligand, et l'on détecte en aval de la seconde paire d'électrodes l'excédent d'analyte que contenait initialement le fluide ou liquide.

Ceci permet alors de pratiquer des dosages semi-quantitatifs : les électrodes négatives captent toutes les Immunoglobulines libres et les électrodes positives, plus petites, captent une partie seulement des complexes Ig + antigène, cette partie des complexes correspondant au taux physiologique d'antigène à ne pas dépasser. S'il y a excès de cet antigène, les électrodes positives ne pourront pas capter tous les complexes formés et un signal sera alors fourni, indiquant l'excès de cet antigène au-dessus des taux physiologiques.

Un avantage important de l'invention est la rapidité du dosage. Entre le dépôt de l'échantillon puis de la solution d'anticorps marqués et l'apparition du résultat, il ne s'écoule guère plus de 5 à 10 secondes. La raison en est que l'attraction ou la répulsion des anticorps est instantanée alors que si cette réaction etait entièrement immunologique (attraction du complexe anticorps+antigène par un second anticorps préalablement fixé par exemple), elle aurait alors nécessité une incubation. En fait, la réaction immunologique se produit dans la feuille poreuse isolante ou électriquement inerte immédiatement après le dépôt. Cette réaction immunologique peut être accélérée de l'ordre de 10.000 fois par l'addition de réactifs appropriés (comme le polyéthylène glycol 8000 par exemple). La réaction qui se produit ensuite entre les électrodes n'est donc plus immunologique mais électrique, donc instantanée, ce qui confère à cette invention une qualité essentielle pour les dosages en urgence (à titre d'exemples, enzymes myocardites dans l'infarctus, annonce d'un embol imminent en dosant le D-Dimère ou les facteurs de la coagulation après la chirurgie osseuse ou dans les phlébites, etc...).

Il est important que les anticorps comportent avant la réaction des groupes NH₂ libres. Il est donc préférable que les anticorps restent monomères et que le marquage se fasse sans production d'agrégats ou de polymères des anticorps mettant en jeu les groupes NH₂ libres. En cas de formation de polymères ou d'agrégats d'anticorps, les sites NH₂ des anticorps agrégés risquent d'être masqués, ce qui réduira d'autant la capacité de leur captage, notamment par des électrodes négatives. Le procédé pourra quand même encore être mis en oeuvre, mais si c'est avec une sensibilité moindre, tant que les anticorps comporteront un nombre suffisant de groupes NH₂ susceptibles d'intervenir dans la réaction antigène-anticorps ou, en l'absence d'antigène, d'être captés de façon suffisamment énergique.

Mais la précédente situation doit de façon très préférée être évitée. En outre, les anticorps marqués (ou autres ligands marqués) doivent être exempts de tout marqueur libre, même sous forme de traces, surtout lorsqu'il s'agit d'une enzyme apte à modifier un substrat (ou groupe de molécules formant substrat). Lors de la fabrication, par exemple, des conjugués anticorps marqués à la peroxydase par des techniques connues, il sera généralement requis de procéder ensuite à une purification des anticorps marqués par plusieurs molécules de peroxydase par des techniques également connues, notamment par chromatographie des produits de réaction pour ne retenir que les pics d'élution contenant les anticorps marqués par plusieurs molécules de peroxydase et d'éliminer tous les autres, a fortiori ceux qui contiennent des molécules de peroxydase libres.

Le marquage des anticorps monomères peut être fait selon un ratio molécules de marquage/par molécule d'anticorps très élevé, pouvant atteindre la valeur de 5. On pourra aussi avoir recours à des mélanges d'anticorps monoclonaux tous marqués, mais reconnaissant des épitopes respectivement distincts de l'antigène à détecter, ce qui accroîtra d'autant plus la sensibilité de la détection.

L'extrême sensibilité du procédé dans nombre de ses applications est due à plusieurs raisons :
- les matériaux utilisés sont tous inertes (matériel poreux ou électrodes) et ne fixent pas la moindre molécule de protéine comme le font les méthodes ELISA responsables d'un bruit de fond important qui interdit les dosages sensibles. Aucune protéine étrangère ou anticorps étranger ne peut être fixé, ce qui entraîne l'absence totale de bruit de fond.
- la zone signal contient les réactifs les plus sensibles et les plus rapides à réagir avec le marquage des anticorps qui y parviennent
- enfin la haute sélectivité des électrodes ne laisse libre aucun des anticorps "piégeable" mais surtout ne piège aucune molécule d'anticorps "non-piégeable" susceptible de fournir le signal. Ce qui fait que le moindre signal ne peut que provenir d'un anticorps marqué et non d'un bruit de fond.

Dans la présente invention, il ne s'agit nullement de séparer ou de faire migrer différemment des poids moléculaires différents ; il s'agit plutôt de capter ou refouler des immunoglobulines selon qu'elles sont liées ou non à leur antigène spécifique. Le poids moléculaire ne joue aucun rôle puisque la technique fonctionne aussi parfaitement avec des fragments Fab issus d'lgG (les plus légères) qu'avec des lgM réputées plus lourdes.

Dans l'invention objet du brevet décrit ici, les électrodes ne supportent aucun réactif. Elles ne font que jouer pendant le court instant où un fluide migre dans la matière poreuse, leur rôle électrique de répulsion ou d'attraction. C'est le résultat de la migration des fluides après le passage entre ces électrodes, qui fournit un signal et non les électrodes.

En variante, le ligand -ou l'analyte- est déposé à sec sur une zone déterminée de la matière poreuse, dans une région essentiellement à l'amont des électrodes, le fluide ou liquide présumé contenir l'analyte -ou le ligand- étant introduit dans cette zone de la matière poreuse.

Par exemple, c'est l'analyte qui est déposé dans la susdite région déterminée essentiellement dans une région en amont des électrodes. Avantageusement, c'est aussi l'analyte qui est porteur de charges électriques, le fluide ou liquide présumé le contenir étant alors introduit dans la région où est déposé le ligand ; après l'éventuelle production *in situ* du complexe, on réintroduit dans la même région une dose distincte de l'analyte lui-même, celui-ci étant cette fois-ci marqué, et l'on détecte la présence éventuelle de l'analyte marqué dans le liquide ayant migré entre les électrodes.

L'invention concerne aussi un dispositif pour la détection d'un analyte susceptible d'être contenu dans un liquide par réaction avec un ligand spécifique de l'analyte pour former un complexe, l'analyte ou le ligand ou les deux à la fois étant porteurs de charges électriques neutralisées ou même inversées dans le complexe lorsque celui-ci est formé, ce dispositif comprenant :
- au moins une paire de deux électrodes pourvues de surfaces qui se font mutuellement face et électriquement chargées, de polarités identiques, ou susceptibles d'être ainsi chargées ;
- une matière poreuse, elle-même électriquement inerte, intercalée entre ces surfaces et autorisant la diffusion en son sein, entre lesdites électrodes, du liquide susceptible d'être déposé dans une zone libre de cette matière poreuse, essentiellement à l'amont de son trajet de migration dans cette matière poreuse ;
- des moyens associés à cette matière poreuse permettant la détection dans le liquide ayant migré dans celle-ci, entre lesdites électrodes, de ceux de ces composants, analyte, ligand ou complexe, qui n'ont pas été captés par les électrodes.

De préférence, les électrodes sont formées de feuilles électrostatiquement chargées ou susceptibles d'être ainsi chargées et que la matière poreuse est elle-même formée d'une feuille prise en sandwich entre les deux électrodes, cette feuille poreuse s'étendant en aval desdites électrodes vis-à-vis du sens de la migration et comportant dans la partie exteme les moyens de détection des constituants ayant migré.

Ce dispositif est avantageusement pourvu de moyens de révélation d'un marqueur porté par l'un des réactifs, par exemple le ligand.

Les moyens associés à la susdite matière ou feuille poreuse électriquement inerte sont eux-mêmes avantageusement constitués par une feuille poreuse en contact avec la première et dans laquelle peut diffuser le liquide ayant migré à travers la première pour venir au contact du révélateur contenu dans cette deuxième feuille.

Dans la description qui suit, on se réfèrera plus particulièrement aux situations apparemment les plus fréquentes auxquelles le procédé et le dispositif de l'invention sont appelés à s'appliquer, celles de la détection d'antigènes particuliers par les anticorps spécifiques déterminés (ou vice-versa).

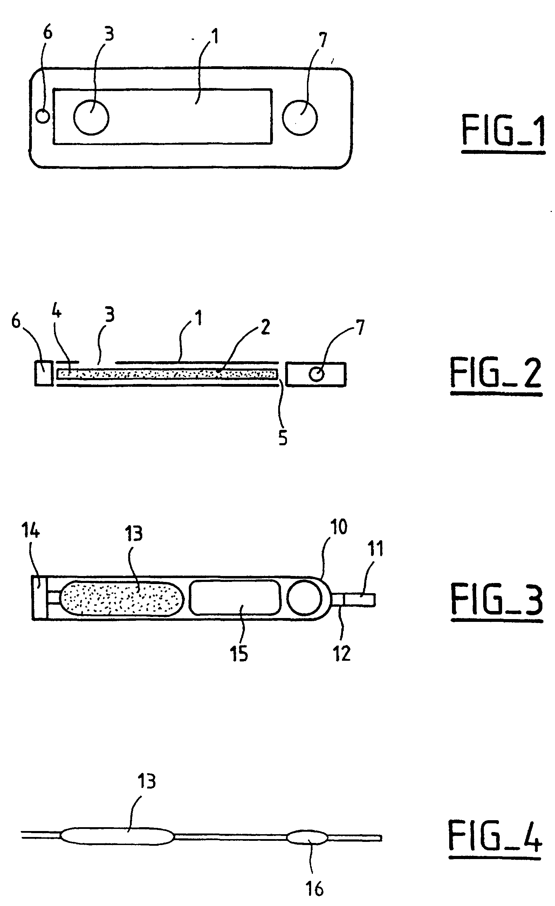

Dans l'un de ses modes de réalisation préférés, le dispositif de l'invention est composé (**figures 1 et 2**) de deux électrodes 1 très fines (lames), séparées par une feuille de matière poreuse 2 électriquement inerte, quelconque (papier, nylon, nitrocellulose, etc...). L'électrode supérieure est perforée (orifice 3) à proximité de l'une de ses extrémités pour laisser apparaître une région de la matière poreuse qui va recevoir dans un premier temps la goutte d'échantillon à tester et dans un deuxième temps la solution d'anticorps ou d'immunoglobulines marqués spécifiques de l'antigène recherché. Cette solution est contenue dans la pipette qui, avantageusement, accompagne le dispositif (**figures 3 et 4**). Dès que la goutte est déposée, elle se comporte comme un contacteur entre les électrodes qui vont alors jouer leur rôle sélectif au fur et à mesure que les fluides migrent entre elles, dans la feuille poreuse et relativement aux électrodes au delà de leur extrémité opposée 5. Les anticorps ou immunoglobulines marquées qui n'ont pas été captés par les électrodes sont alors détectés dans une région 7 dans une matière jouxtant la précédente matière poreuse ou en communication avec elle, dans laquelle peuvent aussi diffuser les fluides, voire même dans une extension de la feuille de matière poreuse, à l'aval des électrodes. Dans la région amont, près de l'orifice 3 où a été déposé l'échantillon, se trouve une petite zone-signal 6 recevant aussi les fluides dans tous les cas (absence ou présence d'antigènes). En se colorant ou en émettant un signal quelconque, elle atteste de la bonne conservation des réactifs et permet dans le cas de mesures réalisées avec un appareillage approprié, d'apprécier la différence entre le taux de marquage avant et après le passage entre les deux électrodes. Cette petite zone-signal peut aussi être au verso du dispositif. Il va de soi que la partie d'électrode qui s'étend entre l'orifice 3 et son extrémité amont est très courte, par exemple de l'ordre du millimètre, de sorte que cette partie ne peut tout au plus capter qu'une proportion minime de l'espèce en cause. Mais il va aussi de soi que l'endroit où s'effectue le dépôt des réactifs peut tout aussi bien être entièrement à l'amont de la paire d'électrodes, de sorte que sera évité toute réaction susceptible de fausser, fut-ce seulement de façon légère, le « contrôle qualité de bonne conservation des réactifs ».

La **figure 1** est donc une vue de dessus et la **figure 2** une vue en coupe du dispositif dans lesquels les chiffres de référence désignent respectivement :
**1 -** Lames d'électrodes chargées positivement.
**2 -** Matière poreuse en contact avec les faces internes des lames 1, pouvant absorber les fluides de l'échantillon pour les diffuser de l'orifice 3 vers les régions 4 et 5,.
**3 -** Orifice dans l'électrode supérieure pour le dépôt de la goutte d'échantillon sur la feuille poreuse.
**4 -** Sortie vers le contrôle qualité de bonne conservation des réactifs.
**5 -** Sortie des fluides après migration entre les lames chargées vers la zone de résultat.
**6 -** Zone-signal du contrôle-qualité.
**7 -** Zone-signal du résultat.

La taille du dispositif peut être du même ordre de grandeur que la puce d'une carte de crédit. Elle peut être insérée dans un boîtier plat ayant approximativement l'épaisseur et le format d'une une carte de crédit. Le boîtier est alors pourvu d'une ouverture à l'aplomb de l'orifice 3 et d'une fenêtre de lecture à l'aplomb de la zone-signal. Dans un autre mode de réalisation de l'invention décrit plus loin, cette ouverture et cette fenêtre peuvent même coïncider. Le dispositif peut aussi être inséré dans une bande destinée à être insérée dans un lecteur quantitatif comme les lecteurs de glycémie. Dans ce cas, le dispositif ne nécessitera pas de source électrique intégrée, puisqu'en l'insérant dans le lecteur juste avant le dépôt de l'échantillon, le dispositif bénéficiera, par l'intermédiaire de deux contacteurs, de l'énergie électrique du lecteur, précisément comme pour les cartes de crédit (dépourvues de source électrique propre à elles). Par ailleurs, la source électrique, en dehors d'une micro-pile classique, peut être plus simplement un micro-capteur de type solaire qui exigera de procéder aux détections ou dosages sous une source lumineuse ou à la lumière. Enfin, certaines électrodes à base d'hydroxyapathite, sont capables d'offrir un puissant électrostatisme propre, sans avoir recours à une source électrique.

Le dispositif peut également être inséré à l'extrémité d'une bande qui sera simplement plongée dans le tube contenant la solution d'anticorps marqués, après dépôt préalable de la gouttelette d'échantillon sur la matière poreuse à travers l'orifice 3. Il va naturellement de soi que dans ce type de dispositif, toutes ses parties, à l'exception de l'emplacement de la masse poreuse auquel l'orifice 3 donne accès, doivent être isolées de façon étanche vis-à-vis de la solution d'anticorps marqués (ou de tous autres ligands marqués).

On peut également former des séries de ces dispositifs, qui seront alors intégrés à des automates capables de traiter ainsi des dizaines d'échantillons en même temps.

Le dispositif de l'invention est avantageusement associé à une pipette -ou micropipette- capable de prélever un volume déterminé de l'échantillon contenant éventuellement l'analyte et à un dispositif apte à délivrer un volume également déterminé du ligand. La micropipette doit être précise, notamment graduée, capable de prélever une certaine quantité mesurée d'échantillon (quelques microlitres) et de la déposer sur la matière poreuse, à travers l'orifice 3 du dispositif.

La micropipette et le dispositif apte à délivrer les anticorps sont avantageusement réunis en un dispositif unique, celui-ci comprenant à l'une de ses extrémités une micro-pipette permettant le prélèvement du volume mesuré du fluide ou liquide contenant éventuellement l'analyte et à son autre extrémité une ampoule ou analogue contenant le volume requis pour la réaction, d'une solution du ligand par exemple d'un anticorps marqué, cette ampoule étant en outre pourvue de moyens de fermeture pouvant être retirés ou brisés de façon contrôlée pour libérer la solution du ligand, après introduction grâce à la pipette du volume de liquide à étudier dans la matière poreuse.

La pipette d'anticorps marqués est en plastique transparent dont le bouchon se casse par simple rotation. Elle contient le volume suffisant de solution d'anticorps pour mouiller, en progressant de l'arrière vers l'avant, la totalité de la matière poreuse de séparation et amener les fluides vers la zone signal. Cette pipette est souvent le seul élément du dispositif à être spécifique de la substance recherchée, puisqu'elle contient les anticorps marqués spécifiques de cette substance. Ce sont ces anticorps qui vont être ou non piégés par les électrodes. Ces dernières sont identiques pour tous les dispositifs et ne supportent aucun réactif chimique ou biologique et encore moins immunologique. Elles sont donc universelles et peuvent être produites en très grandes séries par des procédés robotiques.

La **figure 3** est une vue de dessus et la **figure 4** est une vue de profil d'une telle pipette, notamment une micropipette de prélèvement (10) en une matière plastique souple inapte à fixer des protéines (polypropylène ou polycarbonate) comportant aussi la dose de l'anticorps en l'occurrence. Elle comporte, par exemple, à l'une de ses extrémités, un embout 11 de 1 cm de long et 1 mm de diamètre intérieur, avec un index de volume (trait) 12 et, à son autre extrémité, un compartiment 13 contenant la solution d'anticorps, par exemple 100 µl d'une solution d'anticorps anti-HBs pour un dosage d'antigène HBs (par exemple dans un volume d'environ 4-5 µl d'échantillon à **tester**). Le compartiment 13 est pourvu d'un embout de fermeture 14 qui peut être détaché ou brisé. Une étiquette 15 porte le nom de l'antigène à détecter (par exemple « détection de l'antigène HBs). Un renflement souple 16 (voir **figure 4**) permet de chasser l'air et d'aspirer le volume souhaité d'échantillon à tester. A titre d'exemple la pipette a une longueur de 5 cm et dans sa dimension transversale la plus large, une largeur de 0,8 cm.

Le premier geste sera donc de prélever quelques microlitres d'échantillon à l'aide de la micropipette, de déposer cet échantillon dans l'orifice 3, ensuite de casser par rotation l'embout de fermeture ou bouchon 14 situé à son autre extrémité, du côté de la solution d'anticorps, et enfin de vider cette dernière dans l'orifice 3.

Dans une autre variante, la pipette d'anticorps (sans la micropipette de prélèvement) peut être intégrée à la carte dans une région en matière souple sous forme d'un réservoir qui se déverserait (par pression de doigt par exemple) dans l'orifice 3 ou au contact de la matière poreuse approximativement en cet endroit dès que la gouttelette d'échantillon aurait été déposée à l'aide d'une micropipette fournie séparément.

La zone-signal, enfin, est aménagée dan une pièce de matériau poreux (nylon blanc par exemple) supportant des réactifs susceptibles de transformer la moindre molécule de marqueur des anticorps qui lui parvient, en un signal (colorimétrique ou fluorescent ou chemiluminescent ou radioactif ou autre ). Elle est tout naturellement à l'extrémité des électrodes mais elle peut être n'importe où dans le dispositif, pourvu que les fluides lui parviennent après leur passage entre les électrodes ou sur une étendue suffisante pour leur permettre d'exercer les fonctions qui ont été décrites.

L'invention concerne donc aussi un ensemble de détection immunologique caractérisé par trois éléments distincts :
- un élément en plastique transparent composé :
   - à une extrémité, d'une micropipette pour le prélèvement de quelques microlitres d'échantillon (sang total, sérum, ou tout autre fluide biologique à tester)
   - à l'autre extrémité, d'une pipette contenant la solution d'anticorps marqués devant être déposés à la suite de l'échantillon
- un second élément comprenant les deux électrodes séparées par un matériau inerte, poreux ou fibreux intercalé entre ces électrodes et un élément de matériau poreux imprégné des réactifs et destiné à absorber les fluides à la fin de leur migration entre les électrodes.
- enfin un troisième élément servant de support au précédent. Il peut prendre l'aspect d'une carte de crédit perforée pour recevoir l'échantillon et le contenu de la pipette, ainsi qu'une fenêtre pour montrer la zone-signal. Le second élément serait alors placé sous la carte. Mais ce support peut aussi prendre l'aspect d'une bande à l'extrémité de laquelle se trouve le second élément, afin de l'insérer facilement dans un appareillage lecteur.

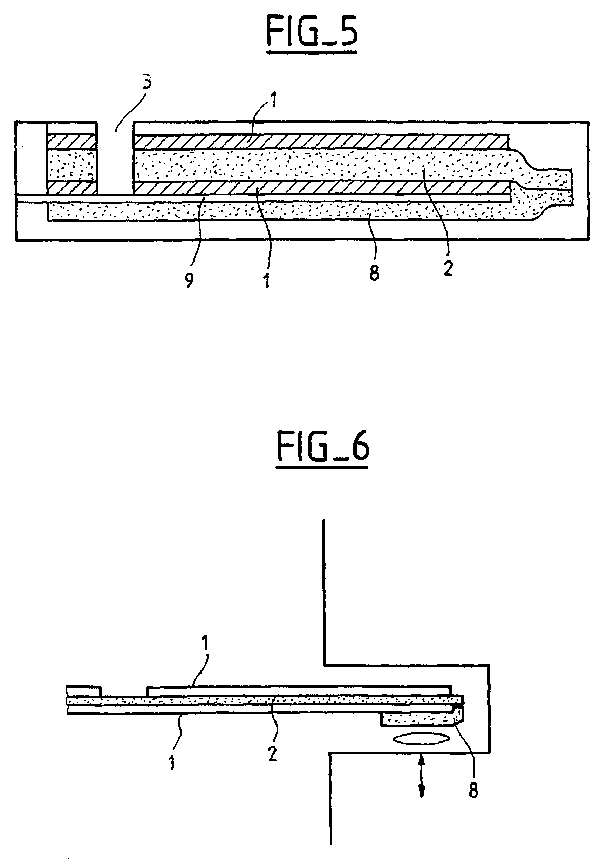

Une variante intéressante est représentée dans la **figure 5**. Selon cette variante, l'orifice 3 qui traverse la première électrode, traverse également la matière poreuse et la deuxième électrode de la même paire, la feuille poreuse étant réunie à l'amont des électrodes à une deuxième feuille poreuse 8 dans laquelle peut diffuser le liquide ayant migré à travers les électrodes, cette seconde feuille étant rabattue sur la face inférieure de la deuxième électrode, à l'opposé de l'entrée de l'orifice, et ce par l'intermédiaire d'une feuille transparente 9, étanche, le cas échéant adhésive, et définissant le fond de l'orifice. La seconde feuille poreuse peut alors être pourvue du révélateur dans la région se trouvant à l'aplomb de l'orifice 3, et cela de façon à permettre la « lecture » de la réaction directement à travers l'orifice dans lequel avaient été introduits les réactifs.

La feuille transparente 9 sert à la fois au colmatage de l'orifice 3 et à l'amenée de la zone-signal sous cette feuille transparente. Les fluides sortis à l'aval des électrodes, sont aspirés par la seconde feuille poreuse, vers cette nouvelle zone signal. La coloration, par exemple, sera vue de dessus, à travers l'adhésif transparent, à l'endroit même de l'orifice 3. Cela simplifie le dispositif qui ne présentera plus qu'un seul orifice pour déposer l'échantillon et permettre à l'utilisateur de voir le résultat dans les quelques secondes qui suivent, à l'endroit même du dépôt. La seule limite à cette intéressante variante concerne les échantillons particulièrement épais, colorés, visqueux, qui peuvent gêner la lecture du résultat par transparence. Mais dans ce cas le dispositif peut être aménagé de façon à permettre à l'utilisateur d'observer le signal à son verso (comme les bandelettes de dosage de la glycémie). On pourrait aussi avoir recours par exemple à un micro-rayon laser, cette variante deviendrait alors très attractive.

Une autre variante encore de dispositif selon l'invention est représentée schématiquement à la **figure 6**, les mêmes éléments étant représentés par les mêmes chiffres de référence. Là encore la zone-signal 7 est présente sur un deuxième élément poreux au contact de la feuille poreuse 2, la zone-signal étant incorporée dans un appareil de quantification permettant la mesure du signal, notamment par l'intermédiaire du degré d'absorption d'un rayon laser.

Les exemples qui suivent n'ont évidemment aucun caractère limitatif. Leur seul but est de faire apprécier la variété des possibilités qu'offre le procédé selon l'invention. Il est évident que l'homme du métier est à même d'en imaginer de nombreuses autres.

### 1 - Détection de l'antigène HBs de l'hépatite B dans le sérum

Matériel : Une pipette de 100 µl d'anticorps monoclonaux marqués Peroxydase concentrés à 2 microgrammes/ml - un dispositif (feuille de papier poreux prise en sandwich entre deux électrodes chargées négativement) dont la zone-signal contient de la tétra-méthyl benzidine, substrat de la Peroxydase.

Protocole : on dépose dans l'orifice 3 un volume de 4 µl de sérum positif en antigène HBs (10 ng/ml), puis les 100 µl d'une solution d'anticorps.

Résultats : 10 secondes après le dépôt, le fluide a diffusé vers la zone-signal et le résultat peut être lu dans la fenêtre de lecture, 5 secondes plus tard, sous la forme d'une coloration bleue pâle qui fonce en un bleu intense en 5 secondes. Total du temps : 20 secondes.

Tests de sensibilité : on dilue le même sérum 1000 fois : nouveau taux d'HBs : 10 picogrammes/ml. On refait la même manipulation : la coloration apparaît dans les mêmes délais, mais légèrement moins intense.

On dilue à nouveau 10 fois : 1 picogramme /ml. Coloration bleue bien visible mais qui a mis 10 secondes de plus pour apparaitre.

Nouvelle dilution de 10 fois : 0,1 picogrammes/ml : coloration bleue mais qui a mis 30 secondes de plus pour être bien visible.

Nouvelle dilution de 10 fois : 10 Millipicogrammes/ml : la coloration est bleue pâle mais elle a mis 1 minute de plus pour apparaitre par rapport au dernier test : total de la durée pour avoir un signal avec 10 millipicogrammes : 2 minutes. (coloration bleue bien visible au bout de 5 minutes)

Nouvelle dilution 10 fois : 1 Millipicogramme/ml. Très discrète coloration après 5 minutes. Ininterprétable à l'oeil nu. Possibilité d'évaluation par appareillage bien étalonné. La sensibilité est extrême : le système parvient à détecter par exemple quelques millipicogrammes d'antigène HBs, soit quelques particules virales par échantillon.

### 2 - Dosage semi-quantitatif du D-Dimère dans le sérum

Le taux physiologique du D-Dimère est de 0,4 microgrammes maximum. Il augmente à la veille de la lyse fibrineuse d'un caillot de sang, annonçant le risque d'une embolie (chirurgie osseuse, pelvienne, cardiologie, etc...). Ce dosage est de type prédictif négatif, c'est-à-dire que 100 % des patients chez qui les tests sont négatifs ne font pas d'embolie alors que 60% des patients chez qui les tests sont positifs risquent de faire une embolie. C'est donc un excellent test d'exclusion du risque d'embolie.

Le problème qui se pose est de savoir comment neutraliser 0,4 µg/ml de D-Dimère. Pour cela on peut avoir recours à une deux paires d'électrodes respectivement appliquées le long de la feuille de matière poreuse, c'est-à-dire une première paire d'électrodes négatives (qui vont capter tous les anticorps non liés à du D-Dimère) puis une deuxième paire de petites électrodes positives (5mm de long) programmées ou calibrées pour capter l'équivalent de 0,4 µg/ml de D-Dimère liés à leurs anticorps monoclonaux.

### Plusieurs sérums vont être testés

a - Sérum A : étalonné à 0,4 µg/ml de D-Dimère.La zone signal se mouille en quelques secondes et aucune coloration ne se produit même au bout de 5 minutes.
b - Sérum B : dosé en laboratoire par Elisa : taux de D-Dimère : 0,76 µg/ml :coloration bleue d'abord pâle en 5 secondes puis bleu bien visible au bout de 20 secondes.
c - Sérum C provenant d'un patient qui venait de faire une embolie pulmonaire : taux de D-Dimère : 5,67 µg/ml : coloration bleue intense, immédiate, 1 seconde après que la zone signal se soit mouillée

### 3 - Détection d'anticorps sériques contre le virus HIV1+2

### Principe :

Le dispositif est, cette fois-ci, préalablement préparé pour ce diagnostic, par dépôt sur la feuille poreuse, à hauteur de l'orifice 3 de peptides du virus VIH, le dépôt ayant été séché avant l'emballage du dispositif. A l'inverse du dosage d'antigènes, qui utilise le même support quel que soit l'antigène recherché (seule varie la pipette d'anticorps marqués spécifique de l'antigène recherché), la détection d'anticorps sériques peut, pour un diagnostic précis, impliquer la préparation préalable de supports spécifiques portant une dose de l'antigène en cause.

La pipette d'anticorps contient 100 µl d'immunoglobulines monoclonales marquées par de la phosphatase alcaline, de très grande affinité et hautement spécifiques des peptides viraux déposés en 3.

La zone signal contient les substrats de la phosphatase alcaline.

### Mode opératoire :

On dépose quelques microlitres de sérum séropositif sur la région de la feuille poreuse comportant l'antigène. Si l'échantillon de sérum est séropositif, les anticorps sériques anti-VIH se complexent rapidement aux peptides viraux. Les anticorps marqués déposés dans un deuxième temps ne trouvent plus de peptides viraux libres et ne seront pas piégés par les électrodes (dans ce cas : Électrodes positives). Ils vont donc migrer vers la zone signal et produiront une coloration attestant de la séropositivité de l'échantillon.

On dilue 1000 fois le sérum séropositif et on recommence : coloration bleue moins intense que la précédente.

On dilue 100 fois cette dernière solution de sérum soit au total une dilution de 100.000 fois : coloration bleue bien visible, mais après 2 minutes.

Si l'échantillon est séronégatif, les quelques microlitres déposés en 3 ne contiennent aucun anticorps sérique. Les peptides viraux préalablement déposés en 3 restent libres. Le dépôt d'anticorps marqués dans un deuxième temps entraîne la formation immédiate de complexes anticorps marqués + peptides viraux, complexes qui seront piégés par des électrodes positives. Il n'y aura aucune coloration de la zone-signal.

Test : total de la durée pour avoir un signal avec 10 millipicogrammes : 2 minutes (coloration bleue bien visible).

Les exemples qui précèdent témoignent donc de l'extrême sensibilité de la détection : 10 millipicogrammes d'antigène ou d'anticorps par millilitre d'échantillon si on utilise une lecture colorimétrique, un millipicogramme si on utilise un appareillage plus sensible que l'oeil humain.

Dans une autre variante encore de l'invention, la matière poreuse comporte des anticorps fixés dans la région d'introduction des réactifs, en amont de la paire d'électrodes, ou au niveau de l'orifice 3, et ce pour permettre la détection cette fois-ci des antigènes correspondants dans un liquide ou fluide à étudier. En l'espèce, les anticorps peuvent être fixés à la matière poreuse, par exemple par l'intermédiaire de la protéine A, dans des conditions ne perturbant pas la fixation des antigènes à détecter.

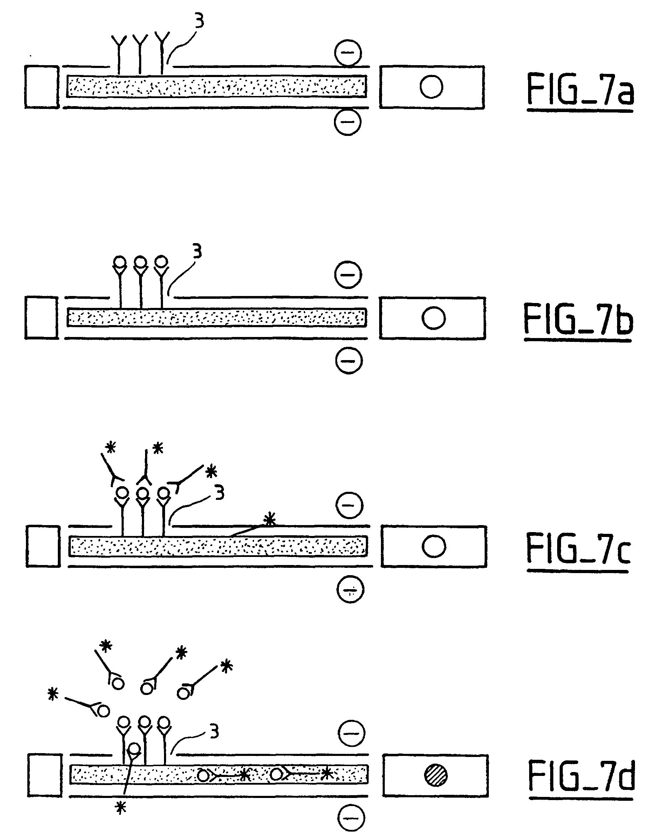

Les schémas de la **figure 7** illustrent les étapes successives d'un dosage semi-quantitatif d'antigènes, qui fait intervenir leur fixation sur les anticorps retenus sur la matière poreuse (**figure 7.1**), puis leur réaction avec d'autres anticorps, cette fois-ci marqués, également spécifiques de l'antigène.

Il s'agit dans l'exemple qui suit, de doser dans un fluide ou liquide donné un éventuel excès d'antigènes vis-à-vis de la normale. Dans cette hypothèse, la quantité d'anticorps retenus dans la région 3 de la feuille poreuse correspond à celle qui permet la fixation de la totalité des antigènes contenus dans un volume déterminé du fluide ou liquide, par exemple un sérum, lorsque la teneur en antigènes de ce fluide n'excède pas une valeur seuil, correspondant par exemple à la normale.

Dans ce dernier cas, la totalité des antigènes apportés par le volume déterminé de sérum va être fixée sur la feuille poreuse (**figure 7.2**).

Les anticorps marqués ensuite ajoutés vont à leur tour se fixer sur les molécules d'antigène retenues sur la matière poreuse, l'éventuel excédent d'anticorps marqués étant alors capté par les électrodes négatives, de sorte qu'aucune détection d'anticorps marqués ne pourra être faite dans la zone-signal 7 (**figure 7.3**). En d'autres termes, l'absence de signal détecté traduira le fait que la teneur en antigène du fluide ou liquide testé ne contenait pas d'antigènes en excès, au delà du seuil déterminé ci-dessus mentionné.

En revanche, si le sérum contient un excès d'antigènes vis-à-vis de la normale, la totalité des antigènes du volume déterminé de liquide déposé dans la région 7 ne peut plus être retenue par les anticorps support de la feuille poreuse. L'addition d'anticorps marqués entraînera alors aussi la production de complexes antigènes-anticorps non retenus dans la région de l'orifice 3. Mais ces complexes ne peuvent plus être captés par les électrodes négatives. Ces complexes antigènes-anticorps en excès marqués diffusent alors dans la matière poreuse et sont détectés dans la zone-signal 7, par exemple grâce à la coloration d'un substrat si le marqueur était de nature enzymatique (**figure 7.4**). En d'autres termes, la détection d'une coloration manifeste l'existence d'un excès d'antigène dans le liquide ou fluide initial dépassant la valeur seuil sus-indiquée.

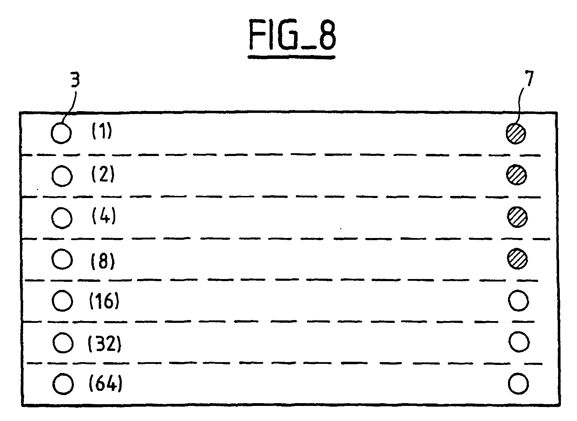

Dans une autre variante, le procédé selon l'invention permet un dosage semi-quantitatif de la teneur en antigènes d'un liquide ou fluide, relativement à des doses déterminées d'anticorps. Le schéma de la **figure 8** illustre l'une des façons de procéder, par exemple grâce à l'utilisation d'un dispositif du genre en question comportant une pluralité de feuilles ou bandelettes poreuses placées côte à côte et coopérant avec des paires d'électrodes négatives correspondantes. A chaque bandelette correspond naturellement aussi une zone de dépôt d'un volume déterminé de la solution d'antigène et une zone-signal pour la détection de l'anticorps marqué engagé dans un complexe antigène-anticorps qui pourrait n'avoir pas été capté par la paire d'électrodes correspondante. Il va naturellement de soi que les bandelettes poreuses des dispositifs parallèles sont isolées latéralement les unes des autres de façon à ce que le liquide diffusant longitudinalement dans l'une d'entre elles vers la zone-signal correspondante, ne puisse pas en même temps diffuser latéralement et envahir les bandelettes poreuses voisines.

Le dosage semi-quantitatif, voire même quantitatif, de la teneur en antigène d'un liquide ou fluide relativement à une dose donnée d'anticorps peut alors être réalisé avec un dispositif de ce type, dès lors que des doses respectivement croissantes d'anticorps auront été fixés à l'origine sur les régions des bandelettes poreuses à l'amont des paires d'électrodes correspondantes (par exemple successivement 1 dose, 2 doses, 4 doses, 8 doses, 16 doses, 32 doses, 64 doses (selon les nombres entre parenthèses dans le schéma de la **figure 8**).

Des volumes déterminés de liquide contenant l'antigène à détecter (le même pour toutes les bandelettes) sont alors déposés dans les régions 3 des différentes bandelettes. Si l'antigène est en excès vis-à-vis de la quantité d'anticorps (1 dose) retenus sur la première bandelette (partie supérieure du schéma de la **figure 8**), seule une partie des antigènes apportés est fixée. Après addition d'anticorps marqués en excès, l'excès d'antigènes non retenus, alors complexés par les anticorps marqués en excès migrent alors vers la zone-signal. Une coloration y est alors constatée. La même observation sera encore faite si la quantité d'antigènes contenus dans le volume déterminé d'échantillon se trouvant encore en excès vis-à-vis des « deux doses d'anticorps retenus sur la deuxième bandelette. On observe pareillement une coloration de la zone-signal correspondante. Il arrive néanmoins un moment où la totalité de l'antigène contenu dans le volume de l'échantillon d'origine a effectivement été fixée sur les anticorps retenus dans les régions 3 des bandelettes correspondantes, ce qui se manifeste alors, même après l'addition d'un fort excès d'anticorps marqués, par la cessation de l'observation d'une coloration dans la zone-signal correspondante. Il en va a fortiori de même dans les bandelettes suivantes dont les régions 3 retenaient dès l'origine des quantités encore plus importantes d'anticorps. Dans le schéma de la **figure 7,** on constate que le seuil d'équilibre entre la teneur en antigène du volume vis-à-vis des quantités croissantes d'anticorps retenus sur les bandelettes se situe entre « huit doses d'anticorps » et « seize doses d'anticorps ».

Il va de soi que ces dernières variantes du procédé selon l'invention sont applicables également au dosage semi-quantitatif d'un analyte vis-à-vis d'un ligand donné, notamment comme cela a été défini dans les revendications 17 à 19. De même l'invention concerne les variantes de dispositifs permettant la mise en oeuvre de ces variantes de procédé. Il est fait particulièrement référence aux variantes de dispositif définies dans les revendications 30 à 32.

## Revendications

1. Procédé pour la détection d'un analyte éventuellement contenu ou susceptible d'être entraîné dans un fluide ou liquide, par réaction avec un ligand spécifique pour former un complexe, l'analyte ou le ligand ou les deux à la fois étant porteurs de charges électriques neutralisées, voire même inversées dans le complexe lorsque celui-ci est formé, **caractérisé en ce que** :
- l'on fait migrer ce fluide ou liquide, après sa mise en contact avec le ligand spécifique, dans une matière poreuse électriquement inerte intercalée entre les faces opposées de deux électrodes électriquement chargées, de même polarité, et
- l'on détecte dans le liquide ayant migré dans la matière poreuse entre les deux électrodes, ceux des composants, analyte, ligand ou complexe qui n'ont pas été captés par les électrodes.

2. Procédé selon la revendication 1, **caractérisé en ce que** les polarités des charges portées par le ligand et celles des électrodes sont de signe contraire, et l'on détecte le complexe dans le liquide ayant migré entre les deux électrodes lorsque le liquide initial contenait l'analyte.

3. Procédé selon la revendication 1, **caractérisé en ce que** les polarités des charges portées par le ligand et celle des électrodes sont de même signe et les polarités des charges portées par l'analyte et le complexe lui-même sont de signe contraire et l'on ne détecte que l'excédent éventuel du ligand initialement mis en oeuvre lorsque l'analyte était initialement présent.

4. Procédé selon l'une quelconque des revendications 1 à 3, **caractérisé en ce que** le ligand porte un marqueur et **en ce que** l'on détecte le marqueur dans une zone signal à laquelle accède le liquide ayant migré dans la matière poreuse, entre les électrodes.

5. Procédé selon la revendication 4, **caractérisé en ce que** le marqueur est transformable en un signal colorimétrique, fluorescent ou chemiluminescent et **en ce que** la zone signal comprend un révélateur du marqueur.

6. Procédé selon la revendication 4 ou la revendication 5, **caractérisé en ce que** la zone signal est elle-même formée dans une matière poreuse en contact avec celle qui est intercalée entre les électrodes, de façon à autoriser l'accès dans la zone signal du liquide ayant migré d'abord dans la matière poreuse intercalée entre les électrodes, puis dans la matière poreuse comportant la zone signal.

7. Procédé selon l'une quelconque des revendications 1 à 6, **caractérisé en ce que** le ligand est déposé à sec sur une zone déterminée de la matière poreuse, dans une région essentiellement à l'amont des électrodes et **en ce que** le fluide ou liquide présumé contenir l'analyte est introduit dans cette zone de la matière poreuse.

8. Procédé selon l'une quelconque des revendications 1 à 7, **caractérisé en ce que** :
- la matière poreuse intercalée entre la précédente paire d'électrodes comporte une extension au delà de celles-ci,
- qu'on la fait passer entre une seconde paire d'électrodes appliquée de part et d'autre de cette extension de la matière poreuse, les électrodes de cette deuxième paire étant également chargées et de polarités identiques entre elles, mais de signe contraire à celui des polarités des électrodes de la précédente paire, et
- **en ce que** l'on effectue la détection en amont de la deuxième paire d'électrodes, après que le liquide ait migré dans la matière poreuse d'abord à travers la première puis à travers la seconde paire d'électrodes dans le cas de dosages semi-quantitatifs.

9. Procédé selon la revendication 8, **caractérisé en ce que** la première paire d'électrodes est d'un signe tel et est calibrée de façon à piéger une quantité maximum prédéterminée de l'analyte sous forme du complexe analyte-ligand, et **en ce que** l'on détecte en aval de la seconde paire d'électrodes l'excédent d'analyte que contenait initialement le fluide ou liquide.

10. Procédé selon la revendication 7, **caractérisé en ce que** l'analyte est porteur de charges électriques, **en ce que** l'on introduit le fluide ou liquide présumé contenir l'analyte dans la région où est déposé le ligand, puis après l'éventuelle production *in situ* du complexe, on réintroduit dans la même région une dose distincte de l'analyte lui-même, celui-ci étant cette fois-ci marqué, et l'on détecte la présence éventuelle de l'analyte marqué dans le liquide ayant migré entre les électrodes.

11. Procédé selon l'une quelconque des revendications 1 à 10, **caractérisé en ce que** l'analyte est constitué par un antigène déterminé, le ligand par l'anticorps spécifique de cet antigène et **en ce que** les électrodes de la première paire d'électrodes ont une polarité négative.

12. Procédé selon la combinaison des revendications 8, 9 et 11, **caractérisé en ce que** la première paire d'électrodes est calibrée de façon à piéger une quantité de complexe éventuellement formé, dans lequel la quantité d'antigène piégée ne peut dépasser celle correspondant à sa teneur normale dans le liquide ou fluide d'origine, en particulier lorsque celui-ci est un fluide biologique provenant d'un sujet sain, et **en ce que** la détection ne porte que sur l'excédent vis-à-vis de la normale de l'antigène éventuellement présent dans le fluide ou liquide d'origine.

13. Procédé selon la revendication 12, **caractérisé par** son application à la détection éventuelle d'un excédent dans le plasma sanguin de D-Dimère.

14. Procédé selon l'une des revendications 1 à 9, **caractérisé en ce que** l'analyte est un anticorps, le ligand un antigène.

15. Procédé selon la revendication 14, **caractérisé en ce que** les polarités de la paire d'électrodes sont de signes contraires à celles de l'anticorps.

16. Procédé selon la combinaison des revendications 10 et 15 pour le diagnostic *in vitro* d'une infection par un HIV dans un fluide biologique.

17. Procédé selon l'une quelconque des revendications 1 à 6, **caractérisé en ce qu'**un volume déterminé contenant l'analyte est déposé dans une zone déterminée de la matière poreuse à l'amont des électrodes, cette zone ne permettant cependant pas la retenue d'une dose d'analyte dépassant une valeur seuil déterminée, **en ce que** l'on dépose ensuite sur cette zone un volume de solution contenant un ligand électriquement chargé, dont la polarité est contraire à celle des électrodes, et **en ce que** l'on détecte le complexe dans le liquide ayant migré entre les deux électrodes dès lors que la quantité d'analyte initialement déposée sur ladite zone aura excédé la dose définie par la valeur seuil déterminée.

18. Procédé **caractérisé par** la mise en jeu d'une pluralité d'ensemble matière poreuse-électrodes et par des répétitions successives du procédé selon la revendication 17 avec le même volume de solution d'analyte dans ces différents ensembles, les zones déterminées des matières poreuses des divers ensembles susdits, sur lesquelles les mêmes volumes de solution d'analyte sont déposés, permettant des retenues maxima d'analyte déterminés correspondant à des valeurs seuil respectivement distinctes, notamment de valeurs respectivement croissantes, et en ce que l'on détermine le rapport de la valeur de seuil par rapport à la teneur de la solution en analyte, au-dessous duquel il n'y a pas de détection et au-dessus duquel il y a détection.

19. Procédé selon la revendication 17 ou 18, **caractérisé en ce que** l'analyte est un antigène et **en ce que** la zone -ou les zones déterminées- de la matière poreuse sont tapissées de quantités d'anticorps respectivement responsables desdites valeurs seuil.

20. Dispositif pour la détection d'un analyte susceptible d'être contenu ou d'être entraîné dans un fluide ou liquide par réaction avec un ligand spécifique de l'analyte pour former un complexe, l'analyte ou le ligand ou les deux à la fois étant porteurs de charges électriques neutralisées ou même inversées dans le complexe lorsque celui-ci est formé, ce dispositif comprenant:
- au moins une paire de deux électrodes (1) pourvues de surfaces qui se font mutuellement face et électriquement chargées, de polarités identiques, ou susceptibles d'être ainsi chargées ;
- une matière poreuse (2), elle-même électriquement inerte, intercalée entre ces surfaces et autorisant la diffusion en son sein, entre lesdites électrodes, du liquide susceptible d'être déposé dans une zone libre de cette matière poreuse, essentiellement à l'amont de son trajet de migration dans cette matière poreuse ;
- des moyens (7) associés à cette matière poreuse permettant la détection dans le liquide ayant migré dans celle-ci, entre lesdites électrodes, de ceux de ces composants, analyte, ligand ou complexe, qui n'ont pas été captés par les électrodes.

21. Dispositif selon la revendication 20, **caractérisé en ce que** les électrodes sont formées de feuilles électrostatiquement chargées ou susceptibles d'être ainsi chargées et que la matière poreuse est elle-même formée d'une feuille prise en sandwich entre les deux électrodes, cette feuille poreuse s'étendant en aval desdites électrodes vis-à-vis du sens de la migration et comportant dans la partie externe les moyens de détection des constituants ayant migré.

22. Dispositif selon la revendication 20 ou 21 pourvu de moyen de révélation d'un marqueur porté par le ligand.

23. Dispositif selon la revendication 21 ou 22, **caractérisé par** une feuille poreuse (8) en contact avec la première et dans laquelle peut diffuser le liquide ayant migré à travers la première pour venir au contact du révélateur contenu dans cette deuxième feuille.

24. Dispositif selon l'une quelconque des revendications 20 à 23, **caractérisé par** un orifice (3) percé dans l'une des électrodes et donnant accès à la matière poreuse dans la région amont de cette électrode, cet orifice étant destiné à l'introduction du fluide ou liquide susceptible de contenir l'analyte et, le cas échéant, du ligand.

25. Dispositif selon la revendication 24 dans lequel le ligand est en fait déjà incorporé à l'état sec à la matière poreuse dans la région même de l'orifice.

26. Dispositif selon la revendication 24, **caractérisé en ce que** l'orifice (3) qui traverse la première feuille, traverse également la matière poreuse (2) et la deuxième électrode de la même paire, la feuille poreuse étant réunie à l'amont des électrodes à une deuxième feuille poreuse (8) dans laquelle peut diffuser le liquide ayant migré à travers les électrodes, cette seconde feuille étant rabattue sur la face inférieure de la deuxième électrode, à l'opposé de l'entrée de l'orifice, et ce par l'intermédiaire d'une feuille transparente (9), le cas échéant adhésive, et définissant le fond de l'orifice, la seconde feuille poreuse étant pourvue du révélateur dans la région se trouvant à l'aplomb de l'orifice (3), et cela de façon à permettre la « lecture » de la réaction directement à travers l'orifice dans lequel avait été introduit l'orifice d'introduction des réactifs dans le dispositif.

27. Dispositif selon l'une quelconque des revendications 20 à 26, en association avec une pipette (10) permettant le prélèvement d'un volume mesuré du fluide ou liquide contenant éventuellement l'analyte et à son autre extrémité une ampoule (13) ou analogue contenant le volume requis pour la réaction d'une solution du ligand, cette pipette étant en outre pourvue de moyens de fermeture pouvant être retirés ou brisés de façon contrôlée pour libérer la solution du ligand, après introduction grâce à la pipette du volume de liquide à étudier dans la matière poreuse.

28. Dispositif selon l'une quelconque des revendications 20 à 26, **caractérisé par** la présence d'un boîtier qui renferme tous les éléments du dispositif, et par la présence, dans ce boîtier même, d'un réservoir de solution du ligand, ce boîtier étant lui-même suffisamment élastique ou flexible pour permettre la libération et l'introduction du contenu du réservoir dans la matière poreuse par simple pression exercée sur les parois du boîtier.

29. Dispositif selon l'une quelconque des revendications 20 à 28 dont toutes les parties sont suffisamment minces et dimensionnées de façon à ce que le tout présente l'apparence d'une carte de crédit.

30. Dispositif selon la revendication 25, **caractérisé en ce que** la zone dans laquelle l'analyte est déposé ne permet cependant la retenue que d'une dose d'analyte ne dépassant pas une valeur seuil déterminée.

31. Dispositif **caractérisé par** un ensemble d'éléments de dispositifs selon la revendication 30, dans lesquels les zones déterminées des matières poreuses permettent des retenues maxima d'analyte déterminées correspondant à des valeurs seuil respectivement distinctes, notamment respectivement croissantes.

32. Dispositif selon la revendication 30 ou 31, **caractérisé en ce que** l'analyte est un antigène et **en ce que** la zone -ou les zones déterminées- de la matière poreuse sont tapissées de quantités d'anticorps respectivement responsables desdites valeurs seuil de retenue des antigènes correspondants.

## Claims

1. Method for the detection of an analyte possibly contained or capable of being carried along in a fluid or liquid, by reaction with a specific ligand to form a complex, the analyte or the ligand or both at the same time being carriers of electrical charges which are neutralised or even reversed in the complex when the latter is formed,
**characterised in that**
- this fluid or liquid, after having been brought into contact with the specific ligand, is made to migrate into an electrically inert, porous material interposed between the opposite faces of two electrically charged electrodes, with the same polarity, and
- in the liquid which has migrated into the porous material between the two electrodes, those of the components, analyte, ligand or complex which have not been picked up by the electrodes are detected.

2. Method according to claim 1, **characterised in that** the polarities of the charges carried by the ligand and those of the electrodes are of opposite sign, and the complex is detected in the liquid which has migrated between the two electrodes when the initial liquid contained the analyte.

3. Method according to claim 1, **characterised in that** the polarities of the charges carried by the ligand and that of the electrodes are of the same sign and the polarities of the charges carried by the analyte and the complex itself are of opposite sign and only the possible excess of the ligand used initially is detected when the analyte was initially present.

4. Method according to any one of claims 1 to 3, **characterised in that** the ligand carries a marker and **in that** the marker is detected in a signal zone to which the liquid has access which has migrated into the porous material, between the electrodes.

5. Method according to claim 4, **characterised in that** the marker can be transformed into a colorimetric, fluorescent or chemiluminescent signal and **in that** the signal zone includes means for revealing the marker.

6. Method according to claim 4 or claim 5, **characterised in that** the signal zone is itself formed in a porous material in contact with the material which is interposed between the electrodes, in such a way as to allow the access into the signal zone of the liquid which has migrated first into the porous material interposed between the electrodes, then into the porous material comprising the signal zone.

7. Method according to any one of claims 1 to 6, **characterised in that** the ligand is deposited in the dry state on a specific zone of the porous material, in a region essentially upstream of the electrodes and **in that** the fluid or liquid presumed to contain the analyte is introduced into this zone of the porous material.

8. Method according to any one of claims 1 to 7, **characterised in that**:
- the porous material interposed between the preceding pair of electrodes includes an extension beyond the latter,
- said material is passed between a second pair of electrodes applied on each side of this extension of the porous material, the electrodes of this second pair being also charged and of identical polarity to each other, but of opposite sign to that of the polarities of the electrodes of the preceding pair, and
- **in that** the detection is carried out upstream of the second pair of electrodes, after the liquid has migrated into the porous material, first through the first pair of electrodes, then through the second pair of electrodes in the case of semi-quantitative dosages.

9. Method according to claim 8, **characterised in that** the first pair of electrodes is of such a sign, and gauged in such a way, as to trap a predetermined maximum quantity of the analyte in the form of the analyte-ligand complex, and **in that** the excess of analyte which the fluid or liquid contained initially is detected downstream of the second pair of electrodes.

10. Method according to claim 7, **characterised in that** the analyte is a carrier of electrical charges, **in that** the fluid or liquid presumed to contain the analyte is introduced into the region where the ligand is deposited, then after the possible production *in situ* of the complex, a distinct dose of the analyte itself is reintroduced into the same region, said analyte being marked this time, and the possible presence of the marked analyte is detected in the liquid which has migrated between the electrodes.

11. Method according to any one of claims 1 to 10, **characterised in that** the analyte is constituted by a defined antigen, the ligand by the specific antibody of this antigen, and **in that** the electrodes of the first pair of electrodes have a negative polarity.

12. Method according to the combination of claims 8, 9 and 11, **characterised in that** the first pair of electrodes is gauged so as to trap a quantity of possibly formed complex, in which the quantity of trapped antigen cannot exceed the quantity corresponding to its normal content in the original liquid or fluid, in particular when the latter is a biological fluid coming from a healthy subject, and **in that** the detection only concerns the excess with regard to the normal quantity of the antigen possibly present in the original fluid or liquid.

13. Method according to claim 12, **characterised by** its application to the possible detection of an excess of D-dimer in the blood plasma.

14. Method according to one of claims 1 to 9, **characterised in that** the analyte is an antibody, the ligand an antigen.

15. Method according to claim 14, **characterised in that** the polarities of the pair of electrodes are of opposite sign to those of the antibody.

16. Method according to the combination of claims 10 and 15 for the diagnosis *in vitro* of an infection by an HIV in a biological fluid.

17. Method according to any one of claims 1 to 6, **characterised in that a** specific volume containing the analyte is deposited in a specific zone of the porous material upstream of the electrodes, this zone not, however, permitting the retention of an analyte dose which exceeds a specific threshold value, **in that** a volume of solution containing an electrically charged ligand is deposited on this zone, the polarity of this ligand being the opposite of that of the electrodes, and **in that** the complex is detected in the liquid which has migrated between the two electrodes once the quantity of analyte initially deposited on said zone exceeds the dose defined by the specific threshold value.

18. Method **characterised by** the use of a plurality of porous material/electrodes assemblies and by successive repetitions of the method according to claim 17 with the same volume of analyte solution in these different assemblies, the specific zones of the porous materials of the above-mentioned various assemblies, on which the same volumes of analyte are deposited, permitting specific maximum retentions of analyte corresponding to respectively distinct threshold values, especially respectively increasing values, and in that the relationship of the threshold value is determined in relation to the analyte content of the solution, below which there is no detection and above which there is detection.

19. Method according to claim 17 or 18, **characterised in that** the analyte is an antigen and **in that** the zone - or specific zones - of the porous material is/are lined with quantities of antibodies respectively responsible for said threshold values.

20. Device for the detection of an analyte capable of being contained or carried along in a fluid or liquid, by reaction with a specific ligand of the analyte to form a complex, the analyte or the ligand or both at the same time being carriers of electrical charges which are neutralised or even reversed in the complex when the latter is formed, this device comprising:
- at least one pair of two electrodes (1) provided with surfaces which face each other and are electrically charged, with identical polarity, or are capable of being thus charged;
- a porous material (2), itself electrically inert, interposed between these surfaces and permitting diffusion into its centre, between said electrodes, of the liquid capable of being deposited in a free zone of this porous material, essentially upstream of its migratory path into this porous material;
- means (7) associated with this porous material permitting the detection in the liquid which has migrated into the latter, between said electrodes, of those of these components, analyte, ligand or complex which have not been picked up by the electrodes.

21. Device according to claim 20, **characterised in that** the electrodes are formed from electro-statically charged sheets or sheets capable of being so charged and that the porous material is itself formed from a sheet sandwiched between the two electrodes, this porous sheet extending downstream of said electrodes with respect to the direction of migration and comprising in the external portion means of detecting the constituents which have migrated.

22. Device according to claim 20 or 21 provided with means of revealing a marker carried by the ligand.

23. Device according to claim 21 or 22, **characterised by** a porous sheet (8) in contact with the first and into which the liquid can diffuse which has migrated through the first to come into contact with the revealing means contained in this second sheet.

24. Device according to any one of claims 20 to 23, **characterised by** an opening (3) pierced in one of the electrodes and giving access to the porous material in the region upstream of this electrode, this opening being intended for the introduction of the fluid or liquid likely to contain the analyte and, if necessary, the ligand.

25. Device according to claim 24 in which the ligand is in fact already incorporated in the dry state in the porous material in the very region of the opening.

26. Device according to claim 24, **characterised in that** the opening (3) which passes through the first sheet, also passes through the porous material (2) and the second electrode of the same pair, the porous sheet being joined upstream of the electrodes to a second porous sheet (8) in which the liquid can diffuse which has migrated through the electrodes, this second sheet being folded down on the lower face of the second electrode, opposite the entrance to the opening, and this through the agency of a transparent sheet (9), if necessary an adhesive sheet, and defining the bottom of the opening, the second porous sheet being provided with the revealing means in the region directly below the opening (3), and this in a manner to permit the "reading" of the reaction directly through the opening into which the opening for introducing reagents into the device had been introduced.

27. Device according to any one of claims 20 to 26, in association with a pipette (10) permitting the withdrawal of a measured volume of the fluid or liquid possibly containing the analyte, and at its other end an ampoule (13) or similar containing the required volume for the reaction of a solution of the ligand, this pipette being moreover provided with closure means which can be removed or broken in a controlled manner to release the solution of the ligand, after the introduction by means of the pipette of the volume of liquid to be studied into the porous material.

28. Device according to any one of claims 20 to 26, **characterised by** the presence of a housing which encloses all the elements of the device and by the presence, in this very housing, of a reservoir of solution of the ligand, this housing being itself sufficiently resilient or flexible to permit the release and the introduction of the contents of the reservoir into the porous material by simple pressure exerted on the walls of the housing.

29. Device according to any one of claims 20 to 28, of which all the parts are sufficiently thin and dimensioned so that the whole has the appearance of a credit card.

30. Device according to claim 25, **characterised in that** the zone in which the analyte is deposited only permits, however, the retention of a dose of analyte which does not exceed a specific threshold value.

31. Device **characterised by** an assembly of elements of devices according to claim 30 in which the specific zones of the porous materials permit specific maximum retentions of analyte corresponding to respectively distinct, especially respectively increasing, threshold values.

32. Device according to claim 30 or 31, **characterised in that** the analyte is an antigen and **in that** the zone - or specific zones - of the porous material are lined with quantities of antibodies respectively responsible for said threshold values for retention of the corresponding antigens.

## Patentansprüche

1. Verfahren zur Erfassung eines Analyts, der gegebenenfalls in einem Fluid oder einer Flüssigkeit enthalten ist oder darin mitgeführt werden kann, durch Reaktion mit einem spezifischen Liganden, um einen Komplex zu bilden, wobei der Analyt oder der Ligand oder beide gleichzeitig Träger von elektrischen Ladungen sind, die in dem Komplex, wenn dieser gebildet ist, neutralisiert oder auch umgekehrt werden, **dadurch gekennzeichnet, daß**:
- man dieses Fluid bzw. diese Flüssigkeit nach seinem bzw. ihrem Kontakt mit dem spezifischen Liganden in eine elektrisch inaktive poröse Substanz wandern läßt, die zwischen den gegenüberliegenden Flächen zweier elektrisch geladener Elektroden mit gleicher Polarität eingefügt ist, und
- man in der Flüssigkeit, die in die poröse Substanz zwischen den beiden Elektroden gewandert ist, diejenigen der Bestandteile, Analyt, Ligand oder Komplex, erfaßt, die nicht durch die Elektroden aufgenommen worden sind.

2. Verfahren nach Anspruch 1, **dadurch gekennzeichnet, daß** die Polaritäten der vom Liganden getragenen Ladungen und diejenigen der Elektroden entgegengesetzte Vorzeichen aufweisen und daß der Komplex in der Flüssigkeit erfaßt wird, die zwischen die beiden Elektroden gewandert ist, wenn die Ausgangsflüssigkeit den Analyt enthielt.

3. Verfahren nach Anspruch 1, **dadurch gekennzeichnet, daß** die Polaritäten der vom Liganden getragenen Ladungen und diejenige der Elektroden das gleiche Vorzeichen aufweisen und die Polaritäten der vom Analyt und vom Komplex selbst getragenen Ladungen entgegengesetzte Vorzeichen aufweisen und daß nur der eventuelle Überschuß des Liganden erfaßt wird, der anfänglich eingesetzt wurde, wenn der Analyt anfänglich vorhanden war.

4. Verfahren nach einem der Ansprüche 1 bis 3, **dadurch gekennzeichnet, daß** der Ligand einen Marker trägt und daß der Marker in einer Signalzone erfaßt wird, zu der die Flüssigkeit gelangt, die in die poröse Substanz zwischen den Elektroden gewandert ist.

5. Verfahren nach Anspruch 4, **dadurch gekennzeichnet, daß** der Marker in ein kolorimetrisches, fluoreszierendes oder chemilumineszierendes Signal umwandelbar ist und daß der Signalbereich einen Entwickler des Markers enthält.

6. Verfahren nach Anspruch 4 oder Anspruch 5, **dadurch gekennzeichnet, daß** die Signalzone selbst in einer porösen Substanz in Kontakt mit derjenigen, die zwischen den Elektroden eingefügt ist, ausgebildet ist, um den Zugang in die Signalzone der Flüssigkeit zu ermöglichen, die zunächst in die zwischen den Elektroden eingefügte poröse Substanz und anschließend in die die Signalzone enthaltende poröse Substanz gewandert ist.

7. Verfahren nach einem der Ansprüche 1 bis 6, **dadurch gekennzeichnet, daß** der Ligand trocken auf einer bestimmten Zone der porösen Substanz in einem Bereich aufgebracht wird, der im wesentlichen vor den Elektroden liegt, und daß das Fluid bzw. die Flüssigkeit, das bzw. die den Analyt enthalten soll, in diese Zone der porösen Substanz eingebracht wird.

8. Verfahren nach einem der Ansprüche 1 bis 7, **dadurch gekennzeichnet, daß**:
- die zwischen dem vorangehenden Elektrodenpaar eingefügte poröse Substanz eine Erweiterung über diese Elektroden hinaus umfaßt,
- daß man sie zwischen einem zweiten Elektrodenpaar hindurchführt, das beiderseits dieser Erweiterung der porösen Substanz angebracht ist, wobei die Elektroden dieses zweiten Elektrodenpaars ebenfalls geladen sind und untereinander identische Polaritäten, aber mit einem dem Vorzeichen der Polaritäten der Elektroden des vorangehenden Elektrodenpaars entgegengesetzten Vorzeichen aufweisen,
- daß die Erfassung vor dem zweiten Elektrodenpaar erfolgt, nachdem die Flüssigkeit in die poröse Substanz zunächst durch das erste und dann durch das zweite Elektrodenpaar im Falle von semiquantitativen Bestimmungen gewandert ist.

9. Verfahren nach Anspruch 8, **dadurch gekennzeichnet, daß** das erste Elektrodenpaar ein so geartetes Vorzeichen aufweist und so kalibriert ist, daß es eine vorbestimmte maximale Menge des Analyts in Form des Analyt-Ligand-Komplexes auffängt und daß hinter dem zweiten Elektrodenpaar der Analytüberschuß erfaßt wird, den das Fluid bzw. die Flüssigkeit anfänglich enthielt.

10. Verfahren nach Anspruch 7, **dadurch gekennzeichnet, daß** der Analyt Träger von elektrischen Ladungen ist, daß das Fluid bzw. die Flüssigkeit, das bzw. die den Analyt enthalten soll, in dem Bereich eingebracht wird, in dem der Ligand aufgebracht ist, und anschließend nach der eventuellen *in situ*-Produktion des Komplexes in den gleichen Bereich eine unterschiedliche Dosis des Analyts selbst eingebracht wird, wobei dieser diesmal markiert ist, und daß das eventuelle Vorhandensein von markiertem Analyt in der Flüssigkeit erfaßt wird, die zwischen die Elektroden gewandert ist.

11. Verfahren nach einem der Ansprüche 1 bis 10, **dadurch gekennzeichnet, daß** der Analyt aus einem bestimmten Antigen und der Ligand aus dem spezifischen Antikörper dieses Antigens besteht und daß die Elektroden des ersten Elektrodenpaars eine negative Polarität aufweisen.

12. Verfahren nach der Kombination der Ansprüche 8, 9 und 11, **dadurch gekennzeichnet, daß** das erste Elektrodenpaar so kalibriert ist, daß eine Menge von gegebenenfalls gebildetem Komplex aufgefangen wird, wobei die aufgefangene Menge Antigen die Menge nicht übersteigen kann, die seinem normalen Gehalt in der ursprünglichen Flüssigkeit bzw. dem ursprünglichen Fluid entspricht, insbesondere wenn es sich dabei um ein von einer gesunden Person stammendes biologisches Fluid handelt, und daß sich die Erfassung nur auf den Überschuß gegenüber dem Normalwert des gegebenenfalls in dem ursprünglichen Fluid bzw. der ursprünglichen Flüssigkeit vorhandenen Antigens bezieht.

13. Verfahren nach Anspruch 12, **gekennzeichnet durch** seine Anwendung auf die eventuelle Erfassung eines Überschusses von D-Dimer im Blutplasma.

14. Verfahren nach einem der Ansprüche 1 bis 9, **dadurch gekennzeichnet, daß** der Analyt ein Antikörper und der Ligand ein Antigen ist.

15. Verfahren nach Anspruch 14, **dadurch gekennzeichnet, daß** die Polaritäten des Elektrodenpaars denen des Antikörpers entgegengesetzte Vorzeichen aufweisen.

16. Verfahren nach der Kombination der Ansprüche 10 und 15 für die *in vitro*-Diagnose einer HIV-Infektion in einem biologischen Fluid.

17. Verfahren nach einem der Ansprüche 1 bis 6, **dadurch gekennzeichnet, daß** ein bestimmtes den Analyt enthaltendes Volumen in einer bestimmten Zone der porösen Substanz vor den Elektroden aufgebracht wird, wobei diese Zone jedoch nicht das Zurückhalten einer Analytdosis ermöglicht, die einen bestimmten Schwellenwert übersteigt, daß anschließend auf dieser Zone ein Lösungsvolumen aufgebracht wird, das einen elektrisch geladenen Liganden enthält, dessen Polarität derjenigen der Elektroden entgegengesetzt ist, und daß der Komplex in der Flüssigkeit erfaßt wird, die zwischen den Elektroden gewandert ist, sobald die anfänglich auf der besagten Zone aufgebrachte Analytmenge die durch den bestimmten Schwellenwert definierte Dosis überschritten hat.

18. Verfahren, **gekennzeichnet durch** den Einsatz einer Mehrzahl von Gruppen aus poröser Substanz und Elektroden und **durch** aufeinanderfolgende Wiederholungen des Verfahrens nach Anspruch 17 mit dem gleichen Volumen Analytlösung in diesen verschiedenen Gruppen, wobei die bestimmten Zonen der porösen Substanzen der verschiedenen vorgenannten Gruppen, auf denen die gleichen Volumina Analytlösung aufgebracht werden, bestimmte maximale Analytzurückhaltungen ermöglichen, die jeweils unterschiedlichen Schwellenwerten entsprechen, insbesondere jeweils ansteigenden Werten, und daß das Verhältnis des Schwellenwerts bezogen auf den Analytgehalt der Lösung bestimmt wird, bei dessen Unterschreitung keine Erfassung und bei dessen Überschreitung eine Erfassung stattfindet.

19. Verfahren nach Anspruch 17 oder 18, **dadurch gekennzeichnet, daß** der Analyt ein Antigen ist und daß die Zone - oder die bestimmten Zonen - der porösen Substanz mit Antikörpermengen überzogen sind, die jeweils für die besagten Schwellenwerte verantwortlich sind.

20. Vorrichtung zur Erfassung eines Analyts, der gegebenenfalls in einem Fluid oder einer Flüssigkeit enthalten ist oder darin mitgeführt werden kann, durch Reaktion mit einem spezifischen Liganden, um einen Komplex zu bilden, wobei der Analyt oder der Ligand oder beide gleichzeitig Träger von elektrischen Ladungen sind, die in dem Komplex, wenn dieser gebildet ist, neutralisiert oder auch umgekehrt werden, wobei diese Vorrichtung folgendes umfaßt:
- wenigstens ein Paar von zwei Elektroden (1) mit Oberflächen, die sich gegenüberliegen und mit identischen Polaritäten elektrisch geladen sind oder so geladen werden können;
- eine selbst elektrisch inaktive poröse Substanz (2), die zwischen diesen Oberflächen eingefügt ist und in ihrem Innern, zwischen den besagten Elektroden, die Diffusion der Flüssigkeit, die in einer freien Zone dieser porösen Substanz aufgebracht werden kann, im wesentlichen vor ihrer Wanderungsbahn in dieser porösen Substanz, ermöglicht;
- mit dieser porösen Substanz verbundene Mittel (7), die in der Flüssigkeit, die in diese, zwischen die Elektroden, gewandert ist, die Erfassung derjenigen dieser Bestandteile, Analyt, Ligand oder Komplex, ermöglicht, die nicht durch die Elektroden aufgefangen worden sind.

21. Vorrichtung nach Anspruch 20, **dadurch gekennzeichnet, daß** die Elektroden aus Folien bestehen, die elektrostatisch geladen sind oder so geladen werden können, und daß die poröse Substanz selbst aus einer Folie besteht, die sandwichartig zwischen den beiden Elektroden eingeschlossen ist, wobei sich diese poröse Folie hinter den besagten Elektroden gegenüber der Wanderungsrichtung erstreckt und in dem äußeren Teil Mittel zur Erfassung der gewanderten Bestandteile umfaßt.

22. Vorrichtung nach Anspruch 20 oder 21, die mit einem Mittel zur Entwicklung eines am Liganden angebrachten Markers versehen ist.

23. Vorrichtung nach Anspruch 21 oder 22, **gekennzeichnet durch** eine poröse Folie (8), die mit der ersten in Kontakt steht und in welche die Flüssigkeit diffundieren kann, die **durch** die erste gewandert ist, um mit dem in dieser zweiten Folie enthaltenen Entwickler in Kontakt zu kommen.

24. Vorrichtung nach einem der Ansprüche 20 bis 23, **gekennzeichnet durch** eine Öffnung (3), die in eine der Elektroden eingearbeitet ist und die Zugang zur porösen Substanz in dem Bereich vor dieser Elektrode gibt, wobei diese Öffnung zur Einführung des Fluids bzw. der Flüssigkeit, das bzw. die den Analyt enthalten kann, und gegebenenfalls des Liganden bestimmt ist.

25. Vorrichtung nach Anspruch 24, in welcher der Ligand faktisch bereits im trockenen Zustand in der porösen Substanz direkt im Bereich der Öffnung eingebunden ist.

26. Vorrichtung nach Anspruch 24, **dadurch gekennzeichnet, daß** die Öffnung (3), die durch die erste Folie hindurchgeht, auch durch die poröse Substanz (2) und die zweite Elektrode des gleichen Paars hindurchgeht, wobei die poröse Folie vor den Elektroden mit einer zweiten porösen Folie (8) verbunden ist, in welche die Flüssigkeit, die durch die Elektroden gewandert ist, diffundieren kann, wobei diese zweite Folie auf der Unterseite der zweiten Elektrode, gegenüber dem Eingang der Öffnung, umgeschlagen ist, und zwar über eine transparente Folie (9), gegebenenfalls eine Haftfolie, die den Boden der Öffnung definiert, wobei die zweite poröse Folie mit dem Entwickler in dem Bereich versehen ist, der sich senkrecht über der Öffnung (3) befindet, und zwar um das "Ablesen" der Reaktion direkt durch die Öffnung zu ermöglichen, in welche die Öffnung zur Einführung der Reagenzien in die Vorrichtung eingeführt worden ist.

27. Vorrichtung nach einem der Ansprüche 20 bis 26 in Verbindung mit einer Pipette (10), die die Entnahme eines abgemessenen Volumens des Fluids bzw. der Flüssigkeit ermöglicht, das bzw. die gegebenenfalls den Analyt enthält, und an ihrem anderen Ende eine Ampulle (13) oder ähnliches, die das erforderliche Volumen für die Reaktion einer Lösung des Liganden enthält, wobei diese Pipette außerdem mit Verschlußmitteln versehen ist, die nach der anhand der Pipette vorgenommenen Einführung des zu untersuchenden Flüssigkeitsvolumens in die poröse Substanz kontrolliert abgezogen oder zerbrochen werden können, um die Lösung des Liganden freizugeben.

28. Vorrichtung nach einem der Ansprüche 20 bis 26, **gekennzeichnet durch** das Vorhandensein eines Gehäuses, das alle Elemente der Vorrichtung umschließt, und das Vorhandensein eines Behälters für die Lösung des Liganden in demselben Gehäuse, wobei dieses Gehäuse selbst ausreichend elastisch oder biegsam ist, um die Freigabe und die Einführung des Inhalts des Behälters in die poröse Substanz **durch** einfachen auf die Wände des Gehäuses ausgeübten Druck zu ermöglichen.

29. Vorrichtung nach einem der Ansprüche 20 bis 28, deren Teile alle ausreichend dünn und so dimensioniert sind, daß das Ganze das Aussehen einer Kreditkarte aufweist.

30. Vorrichtung nach Anspruch 25, **dadurch gekennzeichnet, daß** die Zone, in der der Analyt aufgebracht wird, jedoch nur das Zurückhalten einer Analytdosis ermöglicht, die einen bestimmten Schwellenwert nicht übersteigt.

31. Vorrichtung, **gekennzeichnet durch** eine Gruppe von Elementen von Vorrichtungen nach Anspruch 30, bei denen die bestimmten Zonen der porösen Substanzen bestimmte maximale Zurückhaltungen von Analyt ermöglichen, die jeweils unterschiedlichen, insbesondere jeweils ansteigenden, Schwellenwerten entsprechen.

32. Vorrichtung nach Anspruch 30 oder 31, **dadurch gekennzeichnet, daß** der Analyt ein Antigen ist und daß die Zone - bzw. die bestimmten Zonen - der porösen Substanz Antikörpermengen überzogen sind, die jeweils für die besagten Schwellenwerte für das Zurückhalten der entsprechenden Antigene verantwortlich sind.
